# EUROPÄISCHE PATENTSCHRIFT

(11) **EP 2 796 143 B1**
(45) Veröffentlichungstag und Bekanntmachung des Hinweises auf die Patenterteilung: **27.04.2016**
(21) Anmeldenummer: 13175110.9
(22) Anmeldetag: 04.07.2013
(51) Int. Cl.: A61P 3/04, A61K 36/81, A61K 36/03

(54) **Pharmazeutisches homöopathisches Arzneimittel zur Gewichtsreduktion**
Pharmaceutical homeopathic medication for reducing weight
Médicament homéopathique pharmaceutique pour la réduction du poids

(30) Priorität: 25.04.2013 DE 202013101800 U
(43) Veröffentlichungstag der Anmeldung: 29.10.2014
(73) Patentinhaber: Schnappinger, Uta, 27632 Midlum (DE)
(72) Erfinder: Schnappinger, Uta, 27632 Midlum (DE)
(74) Vertreter: Hansen, Jochen

(56) Entgegenhaltungen:
- FR-A1- 2 839 618
- US-A1- 2013 022 690
- WERK W ET AL: "Helianthus-tuberosus-Therapie bei Uebergewicht. Gewichtsreduktion langfristig stabilisieren! = Helianthus tuberosus in the therapy of obesity. Long-term stabilization of weight reduction", THERAPIEWOCHE, KARLSRUHE, DE, Bd. 44, Nr. 1, 1. Januar 1994 (1994-01-01) , Seiten 34-39, XP009179604, ISSN: 0040-5973

## Beschreibung

Die Erfindung betrifft ein pharmazeutisches homöopathisches Arzneimittel zur Gewichtsreduzierung.

Die Homöopathie ist eine Form der alternativen Medizin. Ärzte oder Homöopathen behandeln Patienten mit stark verdünnten Präparationen, pharmazeutischen Mitteln, die bei gesunden Menschen die Symptome auslösen, die denen der zu behandelnden Patienten ähneln. Mittels dieser Behandlung sollen die Ursachen der Symptome behandelt werden. Im Rahmen der Homöopathie wird der Begriff Mittel für eine Substanz verwendet, die mit einem bestimmten Verfahren hergestellt worden ist und für Patienten Verwendung findet.

Das Grundprinzip der Homöopathie, als das "Gesetz der Ähnlichkeit" bekannt, nämlich "Similia similibus curentur!" also "Ähnliches wird mit Ähnlichem geheilt". Es wurde zuerst von dem deutschen Arzt Samuel Hahnemann im Jahre 1796 angegeben. Homöopathische Mittel werden durch hintereinander geschachtelte Verdünnungen hergestellt, wobei das Schütteln durch kraftvolle markante Schläge auf einem elastischen Körper unterstützt wird. Jede Verdünnung wird durch eine entsprechende Verschüttelung abgeschlossen, um so die Wirkung des pharmazeutischen Mittels zu erhöhen. Diesen Vorgang nennt man Potenzierung. Eine Verdünnung erfolgt oftmals sooft, bis von der ursprünglichen Substanz scheinbar nichts mehr nachgeblieben ist.

Abgesehen von den Symptomen, untersuchen Homöopathen die Aspekte des Patienten in Bezug auf dessen physischen und psychischen Zustand, um im Anschluss mit Hilfe homöopathischer Referenzbücher Abhilfe auf Basis der Gesamtheit der Symptome zu schaffen.

Aus dem Stand der Technik sind unterschiedliche pharmazeutische Mittel und homöopathische Arzneimittel bekannt, um unterschiedliche Wirkungen zu erzielen. Es sind auch pharmazeutische Mittel, insbesondere homöopathische Arzneimittel zur Gewichtsreduzierung bekannt, die dazu beitragen, die Reduktion des Körpergewichtes zu unterstützen. Die bekannten homöopathischen Arzneimittel weisen mehrere Wirkstoffe im Sinne des homöopathischen Wirkstoffbegriffes auf, wobei diese in unterschiedlichen Potenzierungen vorliegen können.
D1 entspricht 1 Teil der ursprünglichen Substanz in 10 Teilen der Lösung,
D2 entspricht 1 Teil der ursprünglichen Substanz in 100 Teilen der Lösung,
D3 entspricht 1 Teil der ursprünglichen Substanz in 1.000 Teilen der Lösung,
D6 entspricht 1 Teil der ursprünglichen Substanz in 1.000.000 Teilen der Lösung,
D9 entspricht 1 Teil der ursprünglichen Substanz in 10⁹ Teilen der Lösung,
D12 entspricht 1 Teil der ursprünglichen Substanz in 10¹² Teilen der Lösung,
D20 entspricht 1 Teil der ursprünglichen Substanz in 10²⁰ Teilen der Lösung,
D23 entspricht 1 Teil der ursprünglichen Substanz in 10²³ Teilen der Lösung.

Die Homöopathie beinhaltet den Prozess der von Homöopathen als "Dynamisierung" oder "Potenzierung" bezeichnet wird. Hierbei wird eine Substanz mit Alkohol oder destilliertem Wasser verdünnt und dann kräftig in einem Prozess der "Verschüttelung" hergestellt. Der Begründer der Homöopathie, Samuel Hahnemann glaubte, dass der Prozess der Verschüttelung die "vitale Energie" der verdünnten Substanz aktiviert wird, und dass aufeinanderfolgende Verdünnungen die "Potenz" des Mittels erhöht.

Das homöopathische Arzneibuch (HAB) ist vergleichbar mit anderen pharmazeutischen Büchern und umfasst einen allgemeinen Teil und einen Teil mit Monographien der Ausgangsstoffe, zusätzlich zu ihren üblichen Qualitätsstandards und den Vorgaben. Mehrere Potenz Skalen sind in der Homöopathie bekannt. Homöopathen entwickelten eine Dezimalskala (D oder Dil.), wobei jede Stufe ein Verdünnen der Substanz um das zehnfache seines ursprünglichen Volumens mit sich bringt. Die hintereinander ausgeführte Verdünnung einer Lösung führt nach jeder Verdünnungsstufe dazu, dass weniger Moleküle der ursprünglichen Substanz pro Liter Lösung vorliegen. Homöopathen sind der Auffassung, dass dieses Wasser eine "wesentliche Eigenschaft" des ursprünglichen Materials bewahrt, weil die Zubereitung nach jeder Verdünnung erschüttert wurde. Es wird angenommen, dass die Dynamisierung oder Verschüttelung der Lösung eine heilende Kraft mit sich bringt, die sich durch Freisetzungen der Substanzen ergibt. Auch wenn die homöopathischen Mittel oft sehr verdünnt vorliegen, sind Homöopathen sicher, dass eine heilende Kraft von diesen homöopathischen Präparaten ausgeht.

Die Prävalenz der Adipositas (BMI ≥ 30) nimmt in Deutschland seit vielen Jahren kontinuierlich zu. Derzeit sind etwa 50 % der erwachsenen Männer mit einen BMI ≥ 25 übergewichtig und ca. 18% mit einem BMI ≥ 30 adipös. Bei den erwachsenen Frauen sind etwa 35 % übergewichtig und knapp 20 % adipös. Auch bei Kindern und Jugendlichen wurde in den letzten Jahren ein Anstieg beobachtet, so dass hier nunmehr kurzfristig Handlungsbedarf besteht.

Der vorliegenden Erfindung liegt die Aufgabe zugrunde, ein pharmazeutisches Mittel, insbesondere homöopathisches Arzneimittel aufzuzeigen, das es ermöglicht, das Körpergewicht zu reduzieren und langfristig auf einem niedrigeren Niveau zu stabilisieren und zu halten, wobei eine wesentlich höhere Wirksamkeit erzielt werden soll. Insbesondere soll im Körper während der Gewichtsreduzierung ein Wohlbefinden bzw. Glücksgefühl hergestellt bzw. aufrecht erhalten werden.

Gelöst wird diese Aufgabe mit einem pharmazeutischen Mittel, insbesondere homöopathischen Arzneimittels zur Gewichtsreduzierung nach Anspruch 1.

Dadurch, dass das pharmazeutische Mittel, insbesondere homöopathisches Arzneimittel zur Gewichtsreduzierung die folgenden Bestandteile und Komponenten mit den jeweiligen Potenzierungen umfasst, nämlich 1 Teil Cactus grandiflorus, in D15 Dil., 1 Teil Berberis aquifolium, in D8 Dil., 1 Teil Capsicum, in D6 Dil., 1 Teil Graphites, in D12 Dil., 2 Teile Fucus vesiculosus, in D6 Dil., 1 Teil Fucus vesiculosus, in D8 Dil., 1 Teil Helianthus tuberosus, in D6 Dil., 1 Teil Kalium chloratum, in D6 Dil. und 1 Teil Natrium phosphoricum, in D6 Dil. ist es möglich, gezielt die Reduzierung des Gewichts zu unterstützen bzw. das Körpergewicht zu reduzieren und gleichzeitig das reduzierte Körpergewicht über eine lange Zeit zu halten. Insbesondere werden alle Körperfunktionen positiv beeinflusst und gesteigert. Die körpereigene Fettverbrennung wird durch das Mittel auf natürlicher, pflanzlicher Basis angekurbelt, die Entschlackung des Körpers gefördert und die Bildung von Endorphinen, den Glückshormonen, angeregt. Insbesondere wird der emotionale Hunger unterdrückt, so dass es nunmehr keinerlei Hungergefühle seitens des Patienten gibt, die auf rein emotionaler Ebene entstehen.

Durch diese Kombination von Wirkstoffen fällt es besonders leicht, insbesondere mit der richtigen Kombination von Nährstoffen, die Körpergewichtsreduzierung durchzustehen. Ein Jo-Jo Effekt wird durch das Mittel effektiv verhindert.

Die Bestandteile des erfindungsgemäßen pharmazeutischen homöopathischen Arzneimittels werden in den letzten beiden Potenzierungsstufen gemeinsam potenziert, um so die bestmögliche Wirkung und Verträglichkeit erzielen zu können sowie besondere Effekte der Wirkstoffe zu erhalten.

Ein weiterer besonderer Vorteil liegt vor, wenn das pharmazeutische Mittel ausschließlich als wässrige Lösung vorliegt. Es wird gänzlich auf Alkohol als Verdünnungsmittel und/oder Trägermedium verzichtet. Es besteht somit keine alkoholische Basis für dieses pharmazeutische Mittel.

Besonders bevorzugt ist Natrium-Chlorid die Trägersubstanz für das pharmazeutische homöopathische Arzneimittel, wobei Natrium-Chlorid in Wasser als Salz gelöst ist.

Mit der hier vorgestellten Methode, einer neuartigen Methode zur Gewichtsreduktion, die mithilfe einer Ernährungsumstellung und homöopathischen Injektionen arbeitet, erreichen die Teilnehmer sowohl eine Gewichtsabnahme über 10% ihres ursprünglichen Ausgangsgewichtes und ebenfalls eine signifikante Reduktion des BMI, des WHtR und des Taillenumfangs. Dadurch verringern sich auch die mit dem Übergewicht einhergehenden kardiovaskulären Risikofaktoren. Zudem bleiben diese Veränderungen über die Zeit konstant und es tritt kein JoJo-Effekt auf.

Die Behandlung muss mindestens 4 Wochen durchgeführt werden. Es gibt hierfür einen genauen Diätplan, der die richtigen Nährstoffe in errechneter Menge enthält. Bei der Durchführung trifft man sich 3x in der Woche zu Einzelterminen, um gemessen und gewogen zu werden. Bei dieser medizinischen Überwachung werden bei jedem Treffen Injektionen des erfindungsgemäßen homöopathischen Mittels unter die Haut gespritzt, die bewirken, dass sich die Verbrennung/ der Stoffwechsel erhöht, der Körper also mehr Energie, trotz niedriger Kalorienaufnahme verbraucht. Daher soll es recht einfach sein, ohne lästige Quälerei und ohne JoJo-Effekt sein Traumgewicht zu erreichen.

Weitere besondere Vorteile des pharmazeutischen Mittels sind insbesondere eine volle Leistungsfähigkeit, ein vermindertes bzw. völlig fehlendes Hungergefühl, ein "gute Laune" Gefühl durch psychische Stabilität, die Stärkung des Selbstwertgefühls, die Verbesserung des Gesundheitszustandes sowie die Steigerung des Wohlbefindens.

Die Gewichtsreduzierung und die anschließende Gewichtsstabilisierung erfolgt im Wesentlichen durch die Aktivierung des Stoffwechsels mit Hilfe des pharmazeutischen Mittels. Durch die arzneimittelwirksamen Bestandteile des pharmazeutischen Mittels erfolgt die Stimulierung zentraler Regulationszentren im Körper. Hierdurch wird der Grundumsatz an Energie erhöht, wodurch der Körper mehr Energie verbraucht.

Nach der Reduzierung des Körpergewichts auf ein gewünschtes Gewicht erfolgt eine Nachbehandlung im Anschluss von lediglich vier Wochen, in der das Mittel weiter eingenommen wird.

Ferner ist eine Ernährung für einen Patienten angegeben, wobei diese bei gleichzeitiger Verwendung des erfindungsgemäßen pharmazeutischen Mittels zur Anwendung kommt. Die Ernährung ist ausgeglichen und genau auf die Körperzusammensetzung und das Körpergewicht des Patienten abgestimmt, wobei die Ernährung nur Eiweiße, Kohlenhydrate, Vitalstoffe und Wasser aufweist und besonders bevorzugt aus 22 Prozent Eiweiß, 1 Prozent Kohlenhydrate, 6 Prozent Vitalstoffen und 70 Prozent aus Wasser.

Zur weiteren Verbesserung sind die Bestandteile in der letzten oder in den letzten beiden Potenzierungen gemeinsam potenziert worden. Insbesondere wird die Wirkung durch das gemeinsame Potenzieren in der letzten bzw. den letzten beiden Potenzierungsschritten gesteigert.

Das offenbarte Verfahren zur Gewichtsreduzierung eines Patienten umfasst die Gabe des erfindungsgemäßen pharmazeutischen Mittels an den Patienten. Hierdurch kann das Körpergewicht in einer sehr hohen und effektiven Weise reduziert werden. Eine Patientin ist in der Lage, ihr Gewicht um ca. 6 bis 8 kg in vier Wochen zu reduzieren. Ein männlicher Patient ist in der Lage sein Körpergewicht um etwa 10 bis 12 Kilogramm in vier Wochen zu reduzieren.

Hierbei erfolgt die Gabe des offenbarte pharmazeutischen Mittels mittels eines Applikators durch den Patienten selbst über einen Nadel-freien transmucosalen Applikator zur Injektion in den Mund des Patienten, wobei jedes mal eine definierte Menge des pharmazeutischen Mittels dem Patienten verabreicht wird. Diese Menge beträgt genau 0,09 ml des erfindungsgemäßen pharmazeutischen Mittels pro Gabe.

Besonders bevorzugt erfolgt die Gabe des offenbarte pharmazeutischen Mittels durch den Patienten selbst oder durch den behandelnden Therapeuten oder Arzt mittels subkutaner Injektion, wobei eine Menge von 1,0 ml alle 2 bis 3 Tage, beispielsweise montags, mittwochs und freitags, erfolgt. Hierbei ist besonders vorteilhaft, dass das erfindungsgemäße pharmazeutische Mittel nicht über die Nahrung zugeführt wird, so dass das offenbarte pharmazeutische Mittel nicht im Magen-Darm-Trakt unerwünscht zersetzt oder verändert wird.

Besonders vorteilhaft ist es, dass neben der Gabe des offenbarte pharmazeutischen Mittels dem Patienten die offenbarte Ernährung zugeführt wird.

Das gesamte Verfahren wird ausschließlich von geschulten Ärzten oder Heilpraktikern durchgeführt und umfasst nur zwei Stufen:
Stufe 1 - Gewichtsverlust, Stufe 2 - Stabilisierung.

In Stufe 1 (Gewichtsverlust) erfolgt das erste Beratungsgespräch, das dazu dient festzustellen, wie viel Gewicht der Patient verlieren will. Der Patient wird gewogen und gemessen. Danach wird dem Patienten ein Ernährungsplan ausgehändigt. Dieser Ernährungsplan ist einfach und in jeder Situation zu folgen. Dreimal in der Woche findet ein Treffen zwischen dem Patienten und dessen Beratung statt, wobei hier das Gewicht des Patienten kontrollieret wird.

Dem Patienten werden eine exakte Berechnung sowie ein entsprechender Ernährungsplan mit den richtigen Nährstoffen in der jeweils richtigen Menge, zum Beispiel Protein in Form von frischem Fleisch, Kohlenhydrate in Form von frischem Gemüse, Salat und Früchte, ausgehändigt.

Es darf über die Ernährung kein zusätzliches Fett aufgenommen werden, da der Körper mit den körpereigenen Fettreserven auskommen und diese aufzehren soll. Zusätzlich werden Vitalstoffe und Wasser in dem Ernährungsplan angeboten. Die Ernährung für den Patienten ist ausgeglichen und genau auf die Körperzusammensetzung und das Körpergewicht des Patienten berechnet und ausgelegt. Die Ernährung besteht nur aus Eiweiß, Kohlenhydrate, Vitalstoffe wie Vitamine und Mineralien und dergleichen sowie aus Wasser.

Stufe 2 (Stabilisierung) beginnt nach dem Erreichen des gewünschten Gewichts. In dieser Phase wird der Patient von seinem persönlichen Berater dazu gebracht, erkennen zu können, wie er oder sie ihre persönlichen Grenzen in Bezug auf Ernährung erkennt und verwaltet. Der Patient lernt, wie man sein Gewicht mit neuen Essgewohnheiten beibehält. Die Verabreichung des Arzneimittels wird jedoch in jedem Fall fortgesetzt, so dass jeder Patient eine weitere Anwendung des Arzneimittels für nur vier Wochen nach dem Erreichen des vorgesehenen Körpergewichts erhält. In Stufe 2 wird die Dosis der pharmazeutischen Zusammensetzung auf eine Anwendung pro Woche reduziert.

Nach dem Vollenden der Stufe 2, die eine minimale und bevorzugte Zeit von vier Wochen nach der Stabilisierung enthält, ist eine dritte Stufe möglich. Die Patienten können Folgesitzungen buchen, so lange diese notwendig erscheinen. Für beste Ergebnisse wird im Folgenden ein Treffen pro Monat für weitere sechs Monate bevorzugt, um das Gewicht der Patienten zu überprüfen.

Es ist auch möglich, noch mehr Gewicht zu verlieren.

Der beste Weg, dieses hohe Maß an Gesundheit zu halten ist, die Behandlung in regelmäßigen Abständen zu wiederholen, zum Beispiel jedes Jahr im Frühjahr oder Herbst.

Nachfolgend wird nochmals abschließend auf die Besonderheiten eingegangen:
Das Besondere liegt in der Kombination der verschiedenen homöopathischen Wirkstoffe deren Wirkungsweise neu ist. Insbesondere dauerte es 15 Jahre, bis diese komplexe Wirkstoffkombination in müheseliger Kleinstarbeit zusammengestellt werden konnte. Immer wieder sind die homöopathischen Inhaltstoffe und deren Potenzen getestet und geändert bzw. optimiert worden bis schließlich das erfindungsgemäße pharmazeutische Mittel in der jetzigen Form vorlag.

Nur die Kombination des erfindungsgemäßen pharmazeutischen Mittels löst folgende positive Reaktionen im menschlichen Organismus aus:
- kein JoJo-Effekt;
- durchschnittliche Gewichtsreduktion in 4 Wochen: 6-8 kg;
- Erhaltung und Steigerung der Leistungsfähigkeit;
- Produktion von Endorphinen (Glückshormonen) daher mit guter Laune abnehmen;
- n u r Fettabbau, Muskelmasse wird nicht angegriffen und bleibt konstant
- zusätzliche Heilenergie wird durch Fettabbau freigesetzt
- signifikante Verbesserung des Gesundheitszustandes bei dem metabolischen Syndrom wie z. B.: Diabetes Mellitus Typ II und sogar Typ I, Normalisierung von Bluthochdruck, weniger autoagressive Reaktionen des Immunsystems wie Nahrungsunverträglichkeiten und Allergien
- Verbesserte Leberfunktionen durch Unterstützung der Entgiftung und somit nachgewiesene Normalisierung von Leberwerten (Gamma-GT) im Blut.
- Oft Verbesserung der Hautreaktionen bei Akne, Schuppenflechte, Neurodermitis und keine, wie sonst oft üblich bei größerer Gewichtsreduktion, entstehenden "Fettschürzen" bzw. übrig gebliebene Hautlappen die operativ entfernt werden müssten. Die Haut bleibt straff.

Mittlerweile konnten weitere Tests durchgeführt werden, wobei das durchschnittliche Gewicht der Teilnehmer zu Beginn der Behandlung 96,58 kg und nach 4 Wochen 87,26 kg betrug. Sie haben durchschnittlich 9,32 kg innerhalb dieser 4 Wochen abgenommen, was einem Gewichtsverlust von ca. 2,33 kg in der Woche entspricht. Minimal wurden 3,6 kg, maximal 15,72 kg in diesem 4-Wochenzeitraum abgenommen. Der BMI von 33,25 und der WHtR (Waist to Height Ratio, Taille-zu-Größe-Verhältnis) von 0,62 zu Beginn der Lipoweg-Methode konnte um ca. 10% auf durchschnittlich 30,04 bzw. 0,57 verringert werden. Männer nehmen hierbei im Durchschnitt 0,5 kg in der Woche mehr ab als Frauen. Diese Veränderungen sind hoch signifikant.

Im Durchschnitt nahmen die Teilnehmer während der ersten 4 Wochen mehr als 10% ihres Ausgangsgewichtes ab. Im weiteren Verlauf konnten die Teilnehmer weiterhin abnehmen und dieses Gewicht halten und z.T. noch weiter abnehmen.

Beim Waist-to-height ratio (WHtR) wird berücksichtigt, dass Bauchfett in der Regel negativere Auswirkungen auf die Gesundheit hat als das Fett, welches an anderen Körperregionen sitzt. Auch hier zeigt die Untersuchung, dass der durchschnittliche WHtR von Beginn der Lipoweg-Methode innerhalb der ersten 4 Wochen um mehr als 10% gesunken ist und im weiteren Verlauf weiter fällt. Auch in Phase 3, der Nachbetreuung nach Beendigung der Kur bis zur erneuten Messung ist der WHtR im Durchschnitt weiterhin gesunken (siehe Abbildung 4). Ebenso ist der Taillenumfang von Beginn der Kur bis zum Zeitpunkt t3 über 10% weniger geworden. Dies ist hoch signifikant.

Insgesamt hat die Methode nicht die alleinige Gewichtsabnahme und Reduktion des Übergewichts als Folge, sondern reduziert auch die viszerale Fettmasse und reduziert damit augenscheinlich die kardiovaskulären Risikofaktoren.

## Patentansprüche

1. Pharmazeutisches homöopathisches Arzneimittel zur Verwendung in der Gewichtsreduzierung, umfassend die folgenden arzneilich wirksamen Bestandteile und Komponenten mit den jeweiligen Potenzierungen:
- 1 Teil Cactus grandiflorus, in D15 Dil.,
- 1 Teil Berberis aquifolium, in D8 Dil.,
- 1 Teil Capsicum, in D6 Dil.,
- 1 Teil Graphites, in D12 Dil.,
- 2 Teile Fucus vesiculosus, in D6 Dil.,
- 1 Teil Fucus vesiculosus, in D8 Dil.,
- 1 Teil Helianthus tuberosus, in D6 Dil.,
- 1 Teil Kalium chloratum, in D6 Dil. und
- 1 Teil Natrium phosphoricum, in D6 Dil..

2. Pharmazeutisches homöopathisches Arzneimittel zur Verwendung nach Anspruch 1, **dadurch gekennzeichnet, dass** die Bestandteile in den letzten beiden Potenzierungsstufen gemeinsam potenziert sind.

3. Pharmazeutisches homöopathisches Arzneimittel zur Verwendung nach Anspruch 1 oder 2, **dadurch gekennzeichnet, dass** das pharmazeutische Mittel als wässrige Lösung vorliegt.

4. Pharmazeutisches homöopathisches Arzneimittel zur Verwendung nach Anspruch 1, 2 oder 3, **dadurch gekennzeichnet, dass** Natrium-Chlorid die Trägersubstanz für das pharmazeutische homöopathische Arzneimittel ist, wobei Natrium-Chlorid in Wasser als Salz gelöst ist.

## Claims

1. A pharmaceutical homeopathic medicinal product for use in weight reduction, comprising the following medically active constituents and components with the respective potentisations:
- 1 part Cactus grandiflorus, in 15X dilution,
- 1 part Berberis aquifolium, in 8X dilution,
- 1 part Capsicum, in 6X dilution,
- 1 part Graphites, in 12X dilution,
- 2 parts Fucus vesiculosus, in 6X dilution,
- 1 part Fucus vesiculosus, in 8X dilution,
- 1 part Helianthus tuberosus, in 6X dilution,
- 1 part Kalium chloratum, in 6X dilution and
- 1 part Natrum phosphoricum, in 6X dilution.

2. The pharmaceutical homeopathic medicinal product for the use according to claim 1, **characterised in that** the constituents are potentised jointly in the last two potentisation steps.

3. The pharmaceutical homeopathic medicinal product for the use according to claim 1 or 2, **characterised in that** the pharmaceutical agent is present as an aqueous solution.

4. The pharmaceutical homeopathic medicinal product for the use according to claim 1, 2 or 3, **characterised in that** sodium chloride is the carrier substance for the pharmaceutical homeopathic medicinal product, wherein sodium chloride is dissolved in water as a salt.

## Revendications

1. Médicament homéopathique pharmaceutique pour l'utilisation dans la réduction de poids, comprenant les substances actives et les composants suivants dans les dilutions indiquées :
- 1 part de Cactus grandiflorus, à la dilution D15,
- 1 part de Berberis aquifolium, à la dilution D8,
- 1 part de Capsicum, à la dilution D6,
- 1 part de Graphites, à la dilution D12,
- 2 parts de Fucus vesiculosus, à la dilution D6,
- 1 part de Fucus vesiculosus, à la dilution D8,
- 1 part de Helianthus tuberosus, à la dilution D6,
- 1 part de Kalium chloratum, à la dilution D6 et
- 1 part de Natrium phosphoricum, à la dilution D6.

2. Médicament homéopathique pharmaceutique pour une utilisation selon la revendication 1, **caractérisé en ce que** les constituants dans les deux dernières dilutions sont dilués ensemble.

3. Médicament homéopathique pharmaceutique pour une utilisation selon la revendication 1 ou 2, **caractérisé en ce que** le produit pharmaceutique se présente sous la forme d'une solution aqueuse.

4. Médicament homéopathique pharmaceutique pour une utilisation selon la revendication 1, 2 ou 3, **caractérisé en ce que** le chlorure de sodium est le support pour le médicament homéopathique pharmaceutique, le chlorure de sodium étant dissout en tant que sel dans l'eau.
